(19) Europäisches Patentamt
European Patent Office
Office européen des brevets

(11) Veröffentlichungsnummer: **0 072 777**
**A2**

# (12) EUROPÄISCHE PATENTANMELDUNG

(21) Anmeldenummer: 82810336.6

(22) Anmeldetag: 10.08.82

(51) Int. Cl.³: C 07 D 213/61

(30) Priorität: 14.08.81 US 292847

(43) Veröffentlichungstag der Anmeldung: 23.02.83 Patentblatt 83/8

(84) Benannte Vertragsstaaten: AT BE CH DE FR GB IT LI NL SE

(71) Anmelder: CIBA-GEIGY AG
Patentabteilung Postfach
CH-4002 Basel(CH)

(72) Erfinder: Fäh, Jansjakob, Dr.
Schlossweg
CH-4466 Ormalingen(CH)

(72) Erfinder: Grieder, Alfred, Dr.
16121, Chantilly avenue
Baton Rouge, LA 70816(US)

## (54) Verfahren zur Herstellung von 2-Chlorpyridinen.

(57) Verfahren zur Herstellung von 2-Chlorpyridinen der Formel I

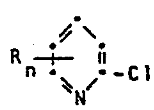

(I),

worin R unabhängig voneinander $C_1$-$C_4$-Alkyl, $C_1$-$C_4$-Halogenalkyl, Halogen oder Carboxy und n 0 oder eine Zahl von 1 bis 4 bedeuten, mit der Massgabe dass höchstens 2 Substituenten R gleichzeitig Alkyl, Halogenalkyl oder Carboxyl bedeuten und dass, wenn R für Chlor und n für 2 stehen, einer der beiden Substituenten nicht die 3- oder 5-Position des Pyridinrings besitzen darf, werden in der Weise durchgeführt, dass man ein 2-Pyridon der Formel II

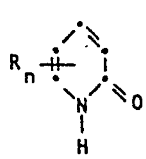

(II),

worin R und n die oben gegebenen Bedeutungen haben, bei 30-150°C in Gegenwart eines N,N-disubstituierten Formamids der Formel III

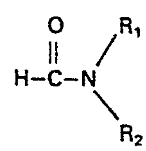

(III),

worin $R_1$ und $R_2$ die gleiche oder verschiedene Bedeutung haben können und für $C_1$-$C_4$-Alkyl stehen oder worin $R_1$ und $R_2$ zusammen mit dem Stickstoffatom einen Pyrrolidin-, Piperidin- oder Morpholinring bilden, und in Gegenwart eines inerten Lösungsmittels mit Phosgen umsetzt. 2-Chlorpyridine sind wertvolle Zwischenprodukte für die Herstellung von herbizid wirksamen α-[4-(Pyrid-2-yloxy)-phenoxy]-alkancarbonsäuren und deren Derivaten.

EP 0 072 777 A2

CIBA-GEIGY AG
Basel (Schweiz)

5-13518/=

Verfahren zur Herstellung von 2-Chlorpyridinen

Die vorliegende Erfindung betrifft ein Verfahren zur Herstellung von 2-Chlorpyridinen.

2-Chlorpyridine sind wertvolle Zwischenprodukte für die Herstellung von herbizid wirksamen α-[4-(Pyrid-2-yloxy)-phenoxy]-alkancarbonsäuren und deren Derivaten. Die Herstellung und Verwendung solcher α-[4-(Pyrid-2-yloxy)-phenoxy]-alkancarbonsäuren und ihrer Derivaten sind zum Beispiel in dem schweizerischen Patent Nr. 622 170 beschrieben.

Die Herstellung von 2-Chlorpyridinen aus N-Alkyl-2-pyridonen mit Phosgen in Toluol als Lösungsmittel oder auch ohne Lösungsmittel ist bekannt. Das Verfahren wird in der Weise durchgeführt, dass man ein N-Alkyl-2-pyridon in eine toluolische Lösung von Phosgen einträgt und das Gemisch in einem geschlossenen System auf 150-180°C erhitzt oder indem man Phosgen in einer Schmelze eines solchen N-Alkylpyridons bei 200°C einleitet. Auf diese Weise werden die Pyridine in einer Ausbeute von 50-90% der Theorie erhalten (Ann. Chem. 486, 71-80, 1931). Die als als Ausgangsmaterialien für dieses Verfahren benötigten N-Alkyl-2-pyridone werden durch Alkylierung der entsprechenden 2-Pyridone erhalten.

Das bekannte Verfahren ist insofern nachteilig, als man die 2-Pyridone zuerst in die N-Alkyl-2-pyridone überführen muss, aus welchen dann bei der nachfolgenden Umsetzung mit Phosgen zu den entsprechenden Pyridinen die N-Alkylgruppe wieder abgespalten wird. Bei diesem Verfahren wird also intermediär eine Gruppe eingeführt, die im Endprodukt gar nicht

vorhanden ist. Dies ist im Hinblick auf die Notwendigkeit der Durchführung einer zusätzlichen Verfahrensstufe und die damit verbundenen Kosten sowie die durch die zusätzliche Verfahrensstufe verursachten Ausbeuteverluste und schliesslich den Verbrauch an Chemikalien, die nicht zum strukturellen Aufbau des Endproduktes beitragen, unwirtschaftlich. Ferner muss wegen der hohen Reaktionstemperatur, bei der das Verfahren durchgeführt wird, in einem geschlossenen System unter Druck oder bei noch höheren Temperaturen, wenn das Verfahren in der Schmelze durchgeführt wird, gearbeitet werden, wodurch sich der im Zusammenhang mit der grosstechnischen Durchführung des Verfahrens notwendige apparative Aufwand beträchtlich erhöht.

Es ist daher das Ziel der vorliegenden Erfindung, die Nachteile der bekannten Arbeitsweise zu vermeiden und ein Verfahren vorzusehen, das die Herstellung von 2-Chlorpyridinen in technischem Massstab auf einfache und wirtschaftliche Weise ermöglicht.

Gemäss vorliegender Erfindung wird daher vorgeschlagen, 2-Chlorpyridine der Formel I

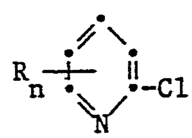

(I),

worin

R unabhängig voneinander $C_1$-$C_4$-Alkyl, $C_1$-$C_4$-Halogenalkyl, Halogen oder Carboxyl und

n Null oder eine Zahl von 1 bis 4 bedeuten mit der Massgabe, dass höchstens zwei Substituenten R gleichzeitig Alkyl, Halogenalkyl oder Carboxyl bedeuten und dass, wenn R für Chlor und n für 2 stehen, einer der beiden Chlorsubstituenten nicht die 3- oder 5-Position des Pyridinrings besetzen darf, in der Weise herzustellen, dass man ein 2-Pyridon der Formel II

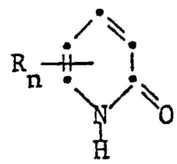

(II),

worin R und n die oben angegebene Bedeutung haben, bei 30-150°C in Gegenwart eines N,N-disubstituierten Formamids der Formel III

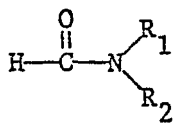

(III),

worin $R_1$ und $R_2$ gleich oder verschieden sein können und jeweils für eine $C_1$-$C_4$-Alkylgruppe stehen oder worin $R_1$ und $R_2$ zusammen mit dem Stickstoffatom einen Pyrrolidin-, Piperidin- oder Morpholinring bilden und in Gegenwart eines inerten Lösungsmittel mit Phosgen umsetzt.

Bevorzugte Verbindungen, die nach dem erfindungsgemässen Verfahren hergestellt werden können, sind die der engeren Formel IV

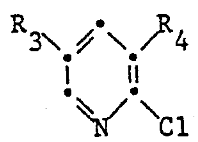

(IV),

worin $R_3$ und $R_4$ unabhängig voneinander für Chlor, Brom, $C_1$-$C_4$-Alkyl oder $C_1$-$C_4$-Halogenalkyl stehen, mit der Massgabe, dass $R_3$ und $R_4$ nicht gleichzeitig Chlor bedeuten.

Weiter bevorzugt sind diejenigen Verbindungen der Formel IV, worin $R_3$ für Brom, Methyl, Ethyl oder $C_1$-$C_2$-Halogenalkyl und $R_4$ für Chlor oder Brom stehen.

Ganz besonders bevorzugt sind diejenigen Verbindungen, worin $R_3$ Methyl oder Trifluormethyl und $R_4$ Chlor oder Brom bedeuten.

Bevorzugte Einzelverbindungen, die nach dem erfindungsgemässen Verfahren hergestellt werden sind 3-Brom-2-chlor-5-methyl-pyridin und

2,2-Dichlor-5-trifluormethyl-pyridin.

Die Umsetzung der 2-Pyridone wird innerhalb des oben angegebenen Temperaturbereichs von 30-150°C, vorzugsweise bei 50-90°C, durchgeführt. Die Umsetzung erfolgt in der Regel bei Normaldruck. Lediglich bei Verwendung eines relativ niedrig-siedenden Lösungsmittels und der Anwendung einer hohen Reaktionstemperatur, beispielsweise im Bereich von 100-150°C, kann es erforderlich sein, in einem geschlossenen System unter Druck zu arbeiten.

Als Lösungsmittel, in dem das erfindungsgemässe Verfahren durchgeführt werden kann, kommen im allgemeinen solche in Betracht, die unter den Reaktionsbedingungen gegenüber den Reaktionsteilnehmern inert sind. Geeignete Lösungsmittel sind insbesondere aromatische Kohlenwasserstoffe wie Benzol, Toluol oder Xylol, aliphatische Kohlenwasserstoffe wie Hexan, Heptan oder Petrolether, cycloaliphatische Kohlenwasserstoffe wie Cyclopentan und Cyclohexan, halogenierte aromatische Kohlenwasserstoffe wie Chlorbenzol oder o-Dichlorbenzol, halogenierte aliphatische Kohlenwasserstoffe wie Methylenchlorid, Chloroform, Tetrachlorkohlenstoff, 1,2-Dichlorethan und Trichlorethylen sowie aliphatische Carbonsäureester wie Ethylacetat und Isopropylacetat. Ein bevorzugtes Lösungsmittel ist Toluol.

Geeignete N,N-disubstituierte Formamide sind N,N-Dimethylformamid, N,N-Diethylformamid, N,N-Dipropylformamid, N,N,-Dibutylformamid, N-Methyl-N-butylformamid sowie N-Formylpyrrolidin, N-Formylpiperidin und N-Formylmorpholin. Ein bevorzugtes N,N-disubstituiertes Formamid ist N,N-Dimethylformamid. Die N,N-disubstituierten Formamide werden in Mengen von 0,01-1,0 Mol pro Mol 2-Pyridon, vorzugsweise 0,05-0,15 Mol pro Mol 2-Pyridon eingesetzt.

Das Phosgen wird erfindungsgemäss in der Regel in mindestens äquimolarer Menge oder in einem Ueberschuss eingesetzt. Zweckmässigerweise

verwendet man 1,0 bis 1,5 Mol Phosgen pro Mol 2-Pyridon. Vorzugsweise setzt man 1,0 bis 1,3 Mol Phosgen pro Mol 2-Pyridon ein. Nach beendigter Umsetzung wird das überschüssige Phosgen durch Zugabe einer wässrigen Lauge wie wässrigen Natronlauge oder wässrigem Ammoniak zerstört. Die Reaktion der 2-Pyridone mit Phosgen nimmt in der Regel 1 bis 5 Stunden in Anspruch und ist in den meisten Fällen nach 2 bis 4 Stunden beendet.

Gemäss einer bevorzugten Ausführungsform des erfindungsgemässen Verfahrens suspendiert man das 2-Pyridon in Toluol, trennt vorhandenes Wasser durch azeotrope Destillation ab, setzt 0,05 bis 0,15 Mol N,N-Dimethylformamid pro Mol 2-Pyridon zu und leitet bei 75-80°C 1,0 bis 1,3 Mol Phosgen pro Mol 2-Pyridon ein. Nach beenidgter Phosgenzugabe lässt man für 1 Stunde bei 75-80°C nachreagieren und zerstört anschliessend das überschüssige Phosgen durch Zugabe von wässrigem Ammoniak. Danach trennt man die Phasen, destilliert das Lösungsmittel aus der organischen Phase ab und gewinnt das 2-Chlorpyridin als Schmelze oder destillierbare Flüssigkeit.

Mit dem erfindungsgemässen Verfahren wird es möglich,2-Chlorpyridine auf einfache und wirtschaftliche Weise in technischem Massstab herzustellen. Das Verfahren vermeidet die nach dem bekannten Verfahren erforderliche N-Alkylierung der 2-Pyridone und kann unter wesentlich milderen Bedingungen durchgeführt werden. Damit wird die Gesamtausbeute gesteigert und der zur Durchführung des Verfahrens notwendige apparative Aufwand verringert.

Das erfindungsgemässe Verfahren wird durch die nachfolgenden Beispiele näher erläutert.

Beispiel 1: In einem 1,5 Liter-Reaktionskolben werden 131,3 g (0,7 Mol) 3-Brom-5-methyl-2-pyridon, 700 ml Toluol und 5,7 g (78 mMol) N,N-Dimethylformamid vorgelegt. Dann wird diese Mischung unter Rühren

auf 75-80°C erwärmt und bei dieser Temperatur 97,4 g (0,98 Mol) Phosgen innerhalb von 2 Stunden eingeleitet. Nach beendigter Zugabe des Phosgens wird für eine Stunde weitergerührt. Danach wird das Reaktionsgemisch auf 15-20°C abgekühlt und mit 84,0 ml Wasser und 60,0 g 30%igem wässrigem Ammoniak verrührt. Nach Abtrennung der wässrigen Phase wird das Toluol bei reduziertem Druck abdestilliert. Als Rückstand erhält man 128,4 g (88,8% d.Th.) 3-Brom-2-chlor-5-methyl-pyridin als Schmelze, die beim Abkühlen erstarrt, Sdp. 63-64°C.

Beispiel 2: In einem 3-Liter-Reaktionsgefäss werden 296,3 g (1,5 Mol) 3-Chlor-5-trifluormethyl-2-pyridon, 1,5 l Toluol und 12,2 g (0,167 Mol) N,N-Dimethylformamid vorgelegt. Diese Mischung wird dann unter gleichzeitigem Rühren auf 75-80°C erhitzt und es werden bei dieser Temperatur 203 g (2,1 Mol) Phosgen innerhalb von 2 bis 3 Stunden eingeleitet. Nach beendigter Zugabe des Phosgens wird noch für 1 Stunde weiter gerührt. Danach wird das Reaktionsgemisch auf 20-25°C abgekühlt und mit 180 ml Wasser und 129 g 30%igem wässrigem Ammoniak verrührt. Nach Abtrennung der wässrigen Phase wird das Toluol bei Normaldruck abdestilliert. Als Rückstand erhält man 305 g (94% d.Th.) 2,3-Dichlor-5-trifluormethyl-pyridin als Oel, Sdp. 76-79°C/55 mb.

Die folgenden Verbindungen werden in analoger Weise hergestellt:

| Verb.Nr. | $R_n$ |
|---|---|
| 1 | 3-Br, 5-$CH_3$ |
| 2 | 3-Cl, 5-$CF_3$ |
| 3 | 3-Cl, 5-$CH_3$ |
| 4 | 3-Br, 5-$CF_3$ |
| 5 | 2,3,6-Cl |
| 6 | 3-Br, 5-Cl |
| 7 | 3,5-Br |
| 8 | 2,4,5,6-Cl |
| 9 | 3-$CH_3$, 3-$CF_3$ |
| 10 | 3-$CF_3$, 5-$CF_3$ |
| 11 | 5-$CF_3$ |
| 12 | 5-$CH_3$ |
| 13 | 3-F, 5-Cl |
| 14 | 5-Cl, 3-F |
| 15 | 3-Cl, 5-COOH |

Patentansprüche

1. Verfahren zur Herstellung eines 2-Chlorpyridins der Formel I

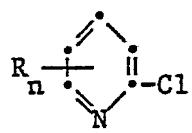

(I),

worin R unabhängig voneinander $C_1$-$C_4$-Alkyl, $C_1$-$C_4$-Halogenalkyl, Halogen oder Carboxyl,
n Null oder eine Zahl von 1 bis 4 bedeuten, mit der Massgabe, dass höchstens 2 Substituenten R gleichzeitig Alkyl, Halogenalkyl oder Carboxyl bedeuten und dass, wenn R für Chlor und n für 2 stehen, einer der Substituenten nicht die 3- oder 5-Position des Pyridinrings besitzen darf, dadurch gekennzeichnet, dass man ein 2-Pyridon der Formel II

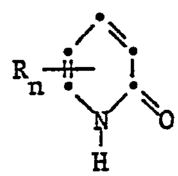

(II),

worin R und n die oben angegebenen Bedeutungen haben, bei 30-150°C in Gegenwart eines N,N-disubstituierten Formamids der Formel III

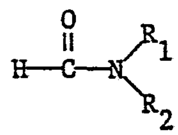

(III),

worin $R_1$ und $R_2$ gleiche oder verschiedene Bedeutung haben können und jeweils für $C_1$-$C_4$-Alkyl stehen, oder worin $R_1$ und $R_2$ zusammen mit dem Stickstoffatom einen Pyrimidin-, Piperidin- oder Morpholinring bilden können, und in Gegenwart eines inerten Lösungsmittels mit Phosgen umsetzt.

2. Verfahren gemäss Anspruch 1 zur Herstellung von Verbindungen der engeren Formel IV

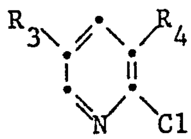

(IV) ,

worin $R_3$ und $R_4$ unabhängig voneinander für Chlor, Brom, $C_1$-$C_4$-Alkyl oder $C_1$-$C_4$-Halogenalkyl stehen, mit der Massgabe, dass $R_3$ und $R_4$ nicht gleichzeitig Chlor bedeuten.

3. Verfahren gemäss Anspruch 2, dadurch gekennzeichnet, dass $R_3$ für Brom, Methyl, Ethyl oder $C_1$-$C_2$-Halogenalkyl und $R_4$ für Chlor oder Brom stehen.

4. Verfahren gemäss Anspruch 2, dadurch gekennzeichnet, dass $R_3$ Methyl oder Trifluormethyl und $R_4$ Chlor oder Brom bedeuten.

5. Verfahren gemäss Anspruch 1, zur Herstellung von 3-Brom-2-chlor-5-methyl-pyridin.

6. Verfahren gemäss Anspruch 1 zur Herstellung von 2,3-Dichlor-5-trifluormethyl-pyridin.

7. Verfahren gemäss Anspruch 1, dadurch gekennzeichnet, dass die Reaktion des 2-Pyridons mit Phosgen bei einer Temperatur von 50-90°C durchgeführt wird.

8. Verfahren gemäss Anspruch 1, dadurch gekennzeichnet, dass als inertes Lösungsmittel ein aromatischer Kohlenwasserstoff, ein aliphatischer Kohlenwasserstoff, ein cycloaliphatischer Kohlenwasserstoff, ein halogenierter aromatischer Kohlenwasserstoff, ein halogenierter aliphatischer Kohlenwasserstoff oder ein aliphatischer Carboxylester verwendet wird.

9. Verfahren gemäss Anspruch 1, dadurch gekennzeichnet, dass das verwendete Lösungsmittel Toluol ist.

10. Verfahren gemäss Anspruch 1, dadurch gekennzeichnet, dass das verwendete N,N-disubstituierte Formamid N,N-Dimethylformamid, N,N-Diethylformamid, N,N-Dipropylformamid, N,N-Dibutylformamid, N-Methyl-N-butylformamid, N-Formylpyrrolidin, N-Formylpiperidin oder N-Formylmorpholin ist.

11. Verfahren gemäss Anspruch 1, dadurch gekennzeichnet, dass das verwendete N,N-disubstituierte Formamid N,N-Dimethylformamid ist.

12. Verfahren gemäss Anspruch 1, dadurch gekennzeichnet, dass das N,N-disubstituierte Formamid in einer Menge von 0,01 bis 1,0 Mol pro Mol 2-Pyridon verwendet wird.

13. Verfahren gemäss Anspruch 1, dadurch gekennzeichnet, dass das N,N-disubstituierte Formamid in einer Menge von 0,05 bis 0,15 Mol pro Mol 2-Pyridon verwendet wird.

14. Verfahren gemäss Anspruch 1, dadurch gekennzeichnet, dass man 1,0 bis 1,5 Mol Phospgen pro Mol 2-Pyridon verwendet.

15. Verfahren gemäss Anspruch 1, dadurch gekennzeichnet, dass man 1,0 bis 1,3 Mol Phosgen pro Mol 2-Pyridon verwendet.

16. Verfahren gemäss Anspruch 1, dadurch gekennzeichnet, dass man das 2-Pyridon in Toluol suspendiert, das anwesende Wasser durch azeotrope Destillation abtrennt, 0,05 bis 0,15 Mol N,N-Dimethylformamid pro Mol 2-Pyridon zusetzt, 1,0 bis 1,3 Mol Phosgen pro Mol 2-Pyridon bei 75-80°C einleitet, das Reaktionsgemisch für eine weitere Stunde rührt, das überschüssige Phosgen durch Zugabe von wässrigem Ammoniak zerstört, die Phasen trennt und aus der organischen Phase nach dem Abtrennen des Lösungsmittels durch Destillation das gebildete 2-Chlorpyridin in Form einer Schmelze oder einer destillierbaren Flüssigkeit gewinnt.